# EUROPEAN PATENT APPLICATION

(11) **EP 2 982 369 A1**
(43) Date of publication of application: **10.02.2016**
(21) Application number: 14779010.9
(22) Date of filing: 31.03.2014
(51) Int. Cl.: A61K 31/192, A61K 9/06, A61P 19/04, A61P 29/00

(54) **PREPARATION FOR TREATING EQUINE INFLAMMATION**

(30) Priority: 01.04.2013 JP 2013075647
(71) Applicant: Daiichi Sankyo Company, Limited, Tokyo 103-8426 (JP)
(72) Inventor: SUZUKI, Yoshinori, Sendai-shi Miyagi 982-0241 (JP)
(74) Representative: Nieuwenhuys, William Francis
(86) International application number: PCT/JP2014/059403
(87) International publication number: WO 2014/163030

(57) **Abstract**

It is an object of the present invention to provide an excellent anti-inflammatory drug for external use that is used for treating inflammation observed in animals (myositis, arthritis, neuritis, tendinitis, tenosynovitis, desmitis, bruise, sprain, etc.), and in particular, for the treatment of bowed tendon observed in horses. The solution of the present invention is an external pharmaceutical composition for preventing or treating inflammation observed in animals, which comprises loxoprofen or a salt thereof as an active ingredient.

## Description

### Technical Field

The present invention relates to an anti-inflammatory drug for external use, which is used for the treatment of inflammation observed in animals (myositis, arthritis, neuritis, tendinitis, tenosynovitis, desmitis, bruise, sprain, etc.), and in particular, for the treatment of bowed tendon observed in horses, and which comprises loxoprofen or a salt thereof as an active ingredient.

### Background Art

As anti-inflammatory drugs for external use that are pharmaceutical products for animals, there have been known methyl salicylate formulations, such as Andress Ointment (Kyoritsu Seiyaku Corporation), Canmel Pasta (Nippon Zenyaku Kogyo Co., Ltd.), Canmel Blue (L) (Nippon Zenyaku Kogyo Co., Ltd.), Pastanogen used for animals "Hokuto" (Hokuto Pharmaceutical Co., Ltd.), and LeitzPasta (Hokuto Pharmaceutical Co., Ltd.). The main ingredient of these formulations is methyl salicylate, and such a methyl salicylate formulation is applied in a suitable amount to the affected area on the skin of an animal such as a bovine, a horse, a pig, a sheep, or a goat (via endermic, diffusion, or transdermal administration, etc.).

Bowed tendon is a condition which involves a partial disruption of tendon fibers of the flexor tendon, which is a tendon connecting the humerus with the elbow ethmoid (wherein, broadly speaking, the flexor tendon consists of two portions, namely, the superficial digital flexor tendon on the outside and the deep digital flexor tendon on the inside), and the affected area often has fever and swelling. Such bowed tendon is commonly seen in a front leg, and also, it develops on the superficial digital flexor tendon more frequently than on the deep digital flexor tendon.

Detailed cause of this disease is still unknown. It is assumed that it could be caused by continuous and repetitive exercise. Also, there is a case in which bowed tendon develops due to inappropriate hoof cutting. Once a horse is affected with this disease, it takes several months to several years until healing is complete. It is rare that the affected area will regain its original strength. It is highly likely that bowed tendon will recur when training or racing starts again. Thus, this disease affects racing performance.

Loxoprofen sodium dihydrate is a phenylpropionic acid-based non-steroidal anti-inflammatory drug (NSAID) represented by the following formula (I): 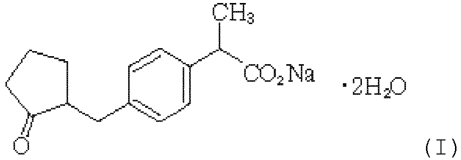 This is a prodrug, which is converted to an active metabolite having strong activity in a living body and then exhibits its activity.

As anti-inflammatory drugs for external use comprising loxoprofen or a salt thereof, LOXONIN(R) PAP (Non Patent Literature 1), LOXONIN(R) TAPE (Non Patent Literature 2), and LOXONIN(R) GEL (Non Patent Literature 3) are known. However, anti-inflammatory drugs for external use comprising loxoprofen or a salt thereof, which are to be administered to animals, have not been known so far.

Some examples are known where an external formulation containing NSAID was used for the treatment of inflammation observed in horses (Patent Literature 1 and Non Patent Literature 4). However, no examples are known where an external formulation containing loxoprofen or a salt thereof was used for the treatment of inflammation observed in horses.

### Citation List

### Patent Literature

Patent Literature 1: WO98/22090

### Non Patent Literature

Non Patent Literature 1: A package insert of the LOXONIN(R) PAP pharmaceutical product (http://www.info.pmda.go.jp/go/pack/2649735S1028_1_06/)
Non Patent Literature 2: A package insert of the LOXONIN(R) TAPE pharmaceutical product (http://www.info.pmda.go.jp/go/pack/2649735S2024_1_04/)
Non Patent Literature 3: A package insert of the LOXONIN(R) GEL pharmaceutical product (http://www.info.pmda.go.jp/go/pack/2649735Q1020_1_03/)
Non Patent Literature 4: Fred J. Caldwell et al., AJVR, Vol. 65, No. 3, March 2004

### Summary of Invention

### Technical Problem

It has been desired to develop an excellent anti-inflammatory drug for external use, which can be used for the treatment of inflammation observed in animals (myositis, arthritis, neuritis, tendinitis, tenosynovitis, desmitis, bruise, sprain, etc.), and in particular, for the treatment of bowed tendon observed in horses. Particularly, once a horse is affected with bowed tendon, it is difficult to treat this disease. Even in a case in which the affected horse has recovered from this disease, it takes a long period of time until healing is complete. Thus, it has been desired to develop a useful therapeutic drug. However, a method for treating bowed tendon observed in horses, which provides sufficient effects, has not yet been established so far. Moreover, when a drug is administered to horses, it is also desired that the administration method is simple.

### Solution to Problem

Under the aforementioned circumstances, the present inventor has focused on the following four points and has conducted intensive studies.
(i) The present formulation is a formulation comprising an active ingredient having a strong anti-inflammatory effect, and fast-acting significant therapeutic effects can be obtained.
(ii) The active ingredient compound itself does not have skin irritancy or damaging properties.
(iii) When the drug is administered, a method capable of easily and simply administering the drug to a subject can be applied.
(iv) The formulation does not cause problems such that the irritancy, skin-damaging properties, odor and the like of the formulation give stress to animals, and as a result, the animals try to remove the drug from feed or do not eat the feed.

As a result of the intensive studies, the present inventor has made the following findings.
(i) The active ingredient is not a compound derived from a counterirritant effect (e.g., methyl salicylate, 1-menthol, etc.), but it is required to be a compound having a mechanism associated with the improvement of inflammation. The compound satisfying this requirement is considered to be a non-steroidal anti-inflammatory drug. Since loxoprofen or a salt thereof has fast-acting strong effects, if it can be optimized to a formulation for external use, it is considered to be suitable as an active ingredient of the present invention. However, the salt of loxoprofen does not have a high transdermal absorption property. Thus, a method of using a carboxyvinyl polymer to convert the salt of loxoprofen into loxoprofen having a high transdermal absorption property is considered to be an optimization means.
(ii) Non-steroidal anti-inflammatory drugs for external use, such as indomethacin and ketoprofen, have skin irritancy and also have a risk of causing skin problems such as contact dermatitis and photosensitivity. Accordingly, it is considered that such a non-steroidal anti-inflammatory drug for external use is not suitable as an active ingredient of the present invention. On the other hand, the salt of loxoprofen is a compound having a low risk of causing such skin problems, and thus, this compound is considered to be suitable as an active ingredient of the present invention.
(iii) When the drug is transdermally administered to an animal, an easy and simple administration method is desired. Accordingly, it is considered that, as an external formulation for animals, a patch is not appropriate, and an embrocation is preferable. However, when the drug is administered to an animal, a large amount of hair covering the body surface thereof becomes an obstacle to application of the drug. Hence, in general, in order to deliver the drug to an affected area, it is necessary to remove the hair covering the affected area and then apply the drug thereto. However, it is not desirable to remove the hair in many cases. In the case of a Vaseline ointment and hydrophilic cream, the body temperature of an animal is hardly transferred to the drug due to the hair of the animal, and the viscosity of the drug is not decreased. As a result, the drug is not allowed to come into contact with the affected area due to the obstacle of the hair, and the drug does not reach the affected area, either. However, as a result of the studies conducted by the present inventor, it was found that, in the present invention in which a carboxyvinyl polymer is used, since the formulation is liquefied by an electrolyte contained in dirt, secretions, etc. adhering to the skin surface of an animal, the drug permeates and reaches the affected area, while the hair of the animal does not become an obstacle. That is to say, it is considered that the present invention relates to a formulation having excellent performance as a drug for use in animals.
(iv) In addition, when indomethacin and ketoprofen are processed into gel formulations for external use, a large amount (50% or more) of ethanol is used, or isopropyl alcohol, which has a dissolving power for the active ingredient and which is able to decrease the mixing concentration but has high irritancy, is used, or a solvent having irritancy (e.g., crotamiton, diisopropyl adipate, etc.) is used. These substances are problematic in that their skin-damaging properties, odor and the like give stress to animals, and as a result, the animals try to remove the drug from feed or do not eat the feed. A great feature of a gel formulation comprising the salt of loxoprofen is that the formulation can be produced using a low concentration (15.0% to 20.0%) of ethanol, and a formulation with low irritancy can be obtained. Hence, animals do not feel uncomfortable with a site to which the drug is applied, and a formulation that does not give stress to the animals can be produced.

Based on the aforementioned findings, the present inventor has found that, when an external pharmaceutical composition comprising loxoprofen or a salt thereof as an active ingredient is used for inflammation observed in animals (preferably, bowed tendon observed in horses, and particularly preferably, bowed tendon observed in thoroughbred horses), it exhibits excellent preventive and/or therapeutic effects, thereby completing the present invention.

Specifically, the present invention relates to:
[1] An external pharmaceutical composition for preventing or treating inflammation observed in animals, which comprises loxoprofen or a salt thereof as an active ingredient.
   The present invention preferably includes the following embodiments.
[2] The external pharmaceutical composition according to [1] above, wherein the loxoprofen or salt thereof is loxoprofen sodium dihydrate.
[3] The external pharmaceutical composition according to [1] or [2] above, which is an endermic formulation.
[4] The external pharmaceutical composition according to [1] or [2] above, which is a gel formulation.
[5] The external pharmaceutical composition according to any one of [1] to [4] above, wherein the animal is a horse (in particular, a thoroughbred horse).
[6] The external pharmaceutical composition according to any one of [1] to [5] above, wherein the inflammation is bowed tendon.
   In addition, the present invention also includes the following embodiments.
[7] A method for preventing or treating inflammation observed in animals, which comprises administering to an affected area an appropriate amount of external pharmaceutical composition comprising loxoprofen or a salt thereof as an active ingredient.
[8] The method for preventing or treating inflammation observed in animals according to [7] above, wherein the loxoprofen or salt thereof is loxoprofen sodium dihydrate.
[9] The method for preventing or treating inflammation observed in animals according to [7] or [8] above, wherein the external pharmaceutical composition is an endermic formulation.
[10] The method for preventing or treating inflammation observed in animals according to [7] or [8] above, wherein the external pharmaceutical composition is a gel formulation.
[11] The method for preventing or treating inflammation observed in animals according to any one of [7] to [10] above, wherein the animal is a horse.
[12] The method for preventing or treating inflammation observed in animals according to any one of [7] to [11] above, wherein the inflammation is bowed tendon.
[13] The method for preventing or treating inflammation observed in animals according to any one of [7] to [12] above, which can be used without causing roughness on the skin.

### Advantageous Effects of Invention

In the present invention, an external pharmaceutical composition comprising loxoprofen or a salt thereof as an active ingredient is used for inflammation observed in animals (preferably, bowed tendon observed in horses, and particularly preferably, bowed tendon observed in thoroughbred horses), so that the composition exhibits excellent preventive and/or therapeutic effects. The external pharmaceutical composition of the present invention is useful because it exhibits preventive and/or therapeutic effects without causing roughness on the skin. In addition, the external pharmaceutical composition of the present invention is also useful because it exhibits preventive and/or therapeutic effects more promptly than conventionally known drugs. Moreover, the external pharmaceutical composition of the present invention is also useful because it is excellent in terms of usability. Animals (in particular, horses (thoroughbred horses)), which are administered with the present external pharmaceutical composition, do not feel stress about administration of the drug, and thus, they do not lick it and do not peel it off. Furthermore, the external pharmaceutical composition of the present invention is also useful because even if animals (in particular, horses (thoroughbred horses)), which are administered with the present external pharmaceutical composition, lick it, and it is thereby placed into the gastrointestinal tract, it hardly causes gastrointestinal tract disturbance.

### Description of Embodiments

The animal defined in the present invention is an animal as a target, to which a pharmaceutical product for animals (a pharmaceutical product used to protect companion animals or livestock animals for food from diseases or parasites) is administered. Examples of the animal in the present invention, which is a small animal, include: mammals such as a dog, a cat, a rabbit, a hamster and a ferret; birds; and reptiles. Examples of the animal in the present invention, which is an industrial animal, include livestock animals (a bovine, a horse, a sheep, a goat, a pig, etc.) and domestic fouls (a chicken, a quail, etc.). Preferred examples of the small animal include mammals such as a dog, a cat, a rabbit, a hamster and a ferret. Preferred examples of the industrial animal include a bovine, a horse, a sheep, a goat and a pig. Particularly preferred examples of the industrial animal are horses, and among horses, thoroughbred horses are particularly preferable.

When the loxoprofen or a salt thereof used in the present invention is left in the air or is subjected to recrystallization, it absorbs moisture and thereby has adsorbed water, so that it becomes a hydrate (e.g. loxoprofen sodium dihydrate) in some cases. Such a hydrate is also included in the loxoprofen or a salt thereof of the present invention.

The amount of the loxoprofen or a salt thereof contained in the external pharmaceutical composition is not particularly limited, as long as it is sufficient for the production of a formulation. For example, the loxoprofen or a salt thereof is used at a weight percentage of preferably 0.1% to 8.0%, and more preferably 0.5% to 5.0%, based on the total weight of the formulation.

Carboxyvinyl polymer used in the present invention is an acrylic acid-based hydrophilic crosslinked polymer, and its molecular weight is not particularly limited. This carboxyvinyl polymer is a component essential for significantly promoting the transdermal absorption of the loxoprofen or a salt thereof.

Upon the formulation of an external pharmaceutical composition, the carboxyvinyl polymer is used in the form of an aqueous solution containing generally 1.0% to 6.0% by weight of, and preferably 2.0% to 4.0% by weight of the carboxyvinyl polymer. The carboxyvinyl polymer is used at a weight percentage of 0.5% to 2.0%, and preferably 0.8% to 1.5%, based on the total weight of the formulation.

The carboxylvinyl polymer has free carboxyl groups, and the free carboxyl groups, together with sodium loxoprofen acting as an active ingredient, are gelatinized, so that a thickening effect can be obtained. This is because sodium ions contained in sodium loxoprofen ionize and gelatinize the free carboxyl groups contained in the carboxyvinyl polymer. During this reaction, sodium loxoprofen is also changed to loxoprofen having high transdermal absorption properties.

The external pharmaceutical composition of the present invention is preferably used in the form of an endermic formulation. Specifically, the present external pharmaceutical composition can be processed into an ointment formulation, a gel formulation, a gel cream formulation, a lotion formulation, or an aerosol formulation. Among these, a gel formulation is preferable.

The inflammation of the present invention means inflammation observed in animals, such as myositis, arthritis, neuritis, tendinitis, tenosynovitis, desmitis, bruise, or sprain. It particularly preferably means bowed tendon observed in horses.

The external pharmaceutical composition of the present invention can be produced, for example, by the following production method.

When the external pharmaceutical composition of the present invention is produced in the form of a gel formulation, a carboxyvinyl polymer is added to loxoprofen sodium dihydrate acting as an active ingredient to be thickened.

As necessary, lower alcohol (e.g. aliphatic alcohols such as methanol, ethanol, propyl alcohol, or isopropyl alcohol, and preferably, ethanol), glycols (e.g. glycerin, propylene glycol, 1,3-butylene glycol, octanediol, ethylene glycol, polyethylene glycol, or D-sorbitol, and preferably, glycerin, propylene glycol, or 1,3-butylene glycol), and water are added, so as to produce a gel formulation.

Further, as necessary, a gel formulation is produced by adding a gel base consisting of another thickener that is different from the carboxyvinyl polymer, a liquid oily substance, an emulsifier, and the like.

As another thickener, a cellulose-based water-soluble polymer compound is preferable. Examples of such a cellulose-based water-soluble polymer compound include methylcellulose, ethylcellulose, hydroxyethylcellulose, hydroxypropylcellulose, and hydroxypropylmethylcellulose. A more preferred cellulose-based water-soluble polymer compound is hydroxypropylcellulose or hydroxypropylmethylcellulose. The most preferred cellulose-based water-soluble polymer compound is hydroxypropylmethylcellulose.

Examples of the liquid oily substance include hydrocarbons such as liquid paraffin, higher fatty acid esters such as isopropyl myristate, and higher alcohols such as octyl dodecanol.

Examples of the emulsifier include glycerin fatty acid ester, sorbitan fatty acid ester, propylene glycol fatty acid ester, and sucrose fatty acid ester.

The amounts of components other than the loxoprofen sodium dihydrate and the carboxyvinyl polymer contained in the external pharmaceutical composition of the present invention are not particularly limited, as long as they are sufficient for the production of a formulation. For instance, lower alcohol is used at a weight percentage of 5.0% to 50.0%, and preferably 10.0% to 30.0%, based on the total weight of the formulation. Glycols are used at a weight percentage of 1.0% to 30.0%, and preferably 5.0% to 20.0%, based on the total weight of the formulation. Water is used at a weight percentage of 40.0% to 90.0%, and preferably 50.0% to 80.0%, based on the total weight of the formulation. A liquid oily substance is used at a weight percentage of 1.0% to 40.0%, and preferably 5.0% to 30.0%, based on the total weight of the formulation. An emulsifier is used at a weight percentage of 0.1% to 10.0%, and preferably 0.5% to 5.0%, based on the total weight of the formulation. A cellulose-based water-soluble polymer compound is used at a weight percentage of 0.1% to 3.0%, and preferably 0.5% to 2.5%, based on the total weight of the formulation. The cellulose-based water-soluble polymer compound and the carboxyvinyl polymer are preferably used, such that the weight percentage of these substances is less than 3.0% based on the total weight of the formulation.

The pH and viscosity of the formulation differ depending on the amounts of thickener and loxoprofen sodium dihydrate exhibiting a neutralization effect on the carboxyvinyl polymer, which are to be contained. In order to adjust pH and viscosity to desired values, a basic substance may be added as appropriate.

Examples of such a basic substance include: inorganic bases such as sodium hydroxide or potassium hydroxide; organic amines such as triethanolamine or diisopropanolamine; and basic amino acids such as arginine, lysine, or ornithine.

Moreover, it is also possible to add a drug effect-enhancing agent as an active ingredient. Examples of such a drug effect-enhancing agent include methyl salicylate, glycol salicylate, 1-menthol, thymol, peppermint oil, nonanoic acid vanillylamide, and capsicum extract.

The anti-inflammatory drug for external use of the present invention may comprise, as appropriate, a suitable active ingredient that can be used in combination, and additives used in common endermic drugs, such as a pH adjuster, an antiseptic, a preservative, an antioxidant, or a stabilizer, as well as the above described components.

The external pharmaceutical composition of the present invention is used by being applied in an appropriate amount to the affected area of an animal that is inflamed.

The number of applications depends on the condition of the affected area. When the affected area has a mild inflammation, the external pharmaceutical composition is applied approximately once every three days. When the affected area has a moderate inflammation, the external pharmaceutical composition is applied several times (1 to 3 times) a day.

The application period (period of use) depends on the condition of the affected area. In general, the external pharmaceutical composition is used for approximately one week, and in some cases, for approximately one month, while following up the course.

Moreover, when the affected area is continuously loaded by exercise or the like, there may be a case in which the external pharmaceutical composition would be used for a long period of time.

There may also be a case in which the external pharmaceutical composition of the present invention would be prophylactically applied to an animal that is inflamed or is likely to become inflamed.

### (Examples)

Hereinafter, the present invention will be described in more detail in the following Examples. However, these Examples are not intended to limit the scope of the present invention.

### (Production Example 1)

Loxoprofen sodium dihydrate (1 g) is dissolved in an aliquot (20 g) of purified water. Separately, 1,3-butylene glycol (5 g) is added to ethanol (20 g), and the mixture is then stirred. Thereafter, hydroxypropylmethylcellulose (0.75 g) is added to the reaction solution, and it is then uniformly dispersed in the solution. To the obtained solution, a 4% carboxyvinyl polymer aqueous solution (22.5 g), triethanolamine (1 g) and the residual purified water (29.7 g) are added, and then, the obtained solution is fully stirred until it becomes homogeneous. To the resulting solution, the above obtained sodium loxoprofen solution is added for gelatinization. The solution is fully stirred until it becomes homogeneous, so as to obtain sodium loxoprofen gel in the form of a colorless transparent jelly. The products of Production Examples 2 to 5 can also be produced by the same method as that applied in Production Example 1.

**[Table 1]**

| Component | Production Example 1 | Production Example 2 | Production Example 3 | Production Example 4 | Production Example 5 |
|---|---|---|---|---|---|
| Loxoprofen sodium dihydrate | 1g | 3g | 2g | 5g | 3g |
| 1-Menthol | - | - | 1g | - | - |
| Methyl salicylate | - | - | - | 0.5g | - |
| Ethanol | 20g | 20g | 15g | 10g | 20g |
| 1,3-Butylene glycol | 5g | 5g | - | - | 5g |
| Glycerin | - | - | 10g | - | - |
| Propylene glycol | - | - | - | 10g | - |
| Hydroxypropylmethylcellulose | 0.75g | 1g | 0.7g | - | 1g |
| Hydroxypropylcellulose | - | - | - | 1. 5g | - |
| 4% carboxyvinyl polymer aqueous solution | 22.5g | 30g | 25g | 30g | 30g |
| Triethanolamine | 1g | - | - | - | 1. 8g |
| Purified water | 49.7g | 41g | 44.7g | 40.5g | 39.2g |

### (Production Example 6)

Loxoprofen sodium dihydrate (3.0 g) is dissolved in purified water (50.0 g). A 4% carboxyvinyl polymer aqueous solution (25.0 g) is added to the obtained solution to gelatinize it, and then, the obtained mixture is fully stirred until it becomes homogeneous. Thereafter, the resultant is heated to 70°C to 80°C in a water bath.

Separately, propylene glycol (10.0 g), octyldodecanol (10.0 g), glyceryl monostearate (1.0 g) and polyoxyl 45 stearate (1.0 g) are heated to 70°C to 80°C in a water bath, and the melted product is then added to the previous gel. The obtained mixture is fully stirred and is then cooled, so as to obtain sodium loxoprofen gel cream in the form of a white cream. The products of Production Examples 7 and 8 can also be produced by the same method as that applied in Production Example 6.

**[Table 2]**

| Component | Production Example 6 | Production Example 7 | Production Example 8 |
|---|---|---|---|
| Loxoprofen sodium dihydrate | 3g | 1g | 0.5g |
| Glycerin | - | 10g | 10g |
| Propylene glycol | 10g | - | - |
| 4% Carboxyvinyl polymer aqueous solution | 25g | 25g | 25g |
| Octyldodecanol | 10g | - | 10g |
| Isopropyl myristate | - | 10g | 5g |
| Glyceryl monostearate | 1g | - | 0.5g |
| Glycol salicylate | - | 2g | 2g |
| Polyoxyl stearate | 1g | - | 0.5g |
| Lauromacrogol | - | 2g | - |
| 1-Menthol | - | - | 3g |
| Triethanolamine | - | - | 1g |
| Purified water | 50g | 50g | 41g |

### (Test Example) Treatment of bowed tendon observed in horses with LOXONIN(R) GEL

### (1) Horses used in test and condition of affected area

Five horses (thoroughbred horses), in all of which the flexor tendon became inflamed and swelled as a result of continuous and repetitive exercise, were used in the test.

### (2) Administration method

An appropriate amount of the above-mentioned "LOXONIN(R) GEL" (Daiichi Sankyo Company, Ltd.) that is a gel formulation containing 1% loxoprofen sodium dihydrate as an active ingredient was applied onto the affected area, followed by massage. The above-described gel formulation was administered at intervals of once every three days, and such administration was continued for 1 month. As a result, swelling caused by inflammation disappeared, and the flexor tendon returned to the same condition as before becoming inflamed.

### (3) Results

From the results of the present test, it was found that significant therapeutic effects can be obtained promptly by administering LOXONIN(R) GEL to horses affected with bowed tendon.

Also, it became clear that LOXONIN(R) GEL is an excellent drug for solving the following problems that may become obstacles in the treatment of bowed tendon observed in horses:
(i) When a methyl salicylate formulation and an indomethacin formulation were applied to horses under the same conditions as those described above, significant effects like those by LOXONIN(R) GEL could not be obtained (recovery of approximately 50%). Moreover, undesired symptoms, such as roughness on the skin that resulted in the removal of hair, or a change in skin color, occurred.
(ii) The methyl salicylate formulation has been problematic in that, since it has certain odor and irritancy, horses dislike it and do not eat feed.
(iii) Oral administration of NSAID was attempted in the treatment of inflammation in horses. However, such oral administration of NSAID has been problematic in that horses are likely to have gastrointestinal tract disturbance, and as a result, they do not eat feed. In the treatment in which LOXONIN(R) GEL was applied onto the affected area, horses did not have such gastrointestinal tract disturbance, and further, there was not a case in which they did not eat feed.

## Claims

1. An external pharmaceutical composition for preventing or treating inflammation observed in animals, which comprises loxoprofen or a salt thereof as an active ingredient.

2. The external pharmaceutical composition according to claim 1, wherein the loxoprofen or salt thereof is loxoprofen sodium dihydrate.

3. The external pharmaceutical composition according to claim 1 or 2, which is an endermic formulation.

4. The external pharmaceutical composition according to claim 1 or 2, which is a gel formulation.

5. The external pharmaceutical composition according to any one of claims 1 to 4, wherein the animal is a horse.

6. The external pharmaceutical composition according to any one of claims 1 to 5, wherein the inflammation is bowed tendon.

7. A method for preventing or treating inflammation observed in animals, which comprises administering to an affected area an appropriate amount of external pharmaceutical composition comprising loxoprofen or a salt thereof as an active ingredient.

8. The method for preventing or treating inflammation observed in animals according to claim 7, wherein the loxoprofen or salt thereof is loxoprofen sodium dihydrate.

9. The method for preventing or treating inflammation observed in animals according to claim 7 or 8, wherein the external pharmaceutical composition is an endermic formulation.

10. The method for preventing or treating inflammation observed in animals according to claim 7 or 8, wherein the external pharmaceutical composition is a gel formulation.

11. The method for preventing or treating inflammation observed in animals according to any one of claims 7 to 10, wherein the animal is a horse.

12. The method for preventing or treating inflammation observed in animals according to any one of claims 7 to 11, wherein the inflammation is bowed tendon.

13. The method for preventing or treating inflammation observed in animals according to any one of claims 7 to 12, which can be used without causing roughness on the skin.
